# EUROPEAN PATENT SPECIFICATION

(11) **EP 1 632 188 B1**
(45) Date of publication and mention of the grant of the patent: **21.10.2009**
(21) Application number: 05018586.7
(22) Date of filing: 26.08.2005
(51) Int. Cl.: A61B 17/22

(54) **Wire for removing intravascular foreign body and medical instrument**
Draht zur Entfernung intravaskulärer Fremdkörper und medizinisches Instrument
Fil vasculaire servant à enlever un corps étranger et un instrument médical

(30) Priority: 07.09.2004 JP 2004260098
(43) Date of publication of application: 08.03.2006
(73) Proprietor: TERUMO KABUSHIKI KAISHA, Tokyo (JP)
(72) Inventor: Kanamaru, Takeshi, Terumo Kabushiki Kaisha, Ashigarakami-gun Kanagawa 259-0151 (JP)
(74) Representative: TBK-Patent

(56) References cited:
- EP-A- 1 273 268
- WO-A-96/23446
- US-A1- 2003 109 889
- US-A1- 2003 225 419

## Description

### BACKGROUND OF THE INVENTION

The present invention relates to a wire for removing an intravascular foreign body (such as embolus in a vessel) and a medical instrument.

The vital statistics of population published by the Ministry of Health, Labor, and Welfare indicates that cancer dominates in the cause of Japanese death and heart disease and cerebral apoplexy come second and third. The increasing deaths and sequelae due to cerebral apoplexy urgently demand to establish its therapeutic method.

A recent development in therapy of cerebral apoplexy is thrombolysis that employs a thrombolitic agent to cure brain infarction in its acute phase. It is effective but its effectiveness is limited. That is, the thrombolitic agent takes a long time for thrombolysis or produces smaller thrombi that scatter to form new emboli. In addition, it has been found that some emboli are insoluble by treatment with a thrombolitic agent.

It has been proved in the US and Europe that the probability to save lives and reduce sequelae would be high if the blood flow is resumed within 3 hours after the onset of cerebral apoplexy. Thus, there is a strong demand for development of a new medical instrument that can be inserted into a cerebral vessel to remove the thrombus directly. An example of such a medical instrument is a wire for removing an intravascular foreign body, which is disclosed in Japanese Patent Laid-open No. 2004-16668 and US 2003/225419.

The wire for removing an intravascular foreign body consists of a wire proper, two branch wires (which branch out from the wire proper), and a plurality of filaments connecting the branch wires. The branch wires and the filaments form a space in which an intravascular foreign body is captured.

The disadvantage of the wire for removing an intravascular foreign body mentioned above is that it sometimes fails to capture an intravascular foreign body depending on its size.

For example, if the intravascular foreign body is smaller than the space in which it is to be captured, it might slip off through the gap between the filaments. In this case, it would be necessary to exchange the wire for removing an intravascular foreign body that matches the size of the foreign body to be captured. This is troublesome.

### SUMMARY OF THE INVENTION

It is a main object of the present invention to provide a wire to surely capture and remove an intravascular foreign body. It is another object of the present invention to provide a medical instrument.

The above-mentioned object of the present invention is achieved by a wire comprising the features of the attached claim 1. Further developments are the subject matter of the depending claims. A medical instrument according to the present invention is stated in claim 9.

In particular, the present invention discloses:
(1) A wire for removing an intravascular foreign body which includes a flexible wire body, at least two branch parts branching out from the forward end of the wire body, and a plurality of filaments extending across the branch part, the branch parts and the filaments forming a space for capturing a foreign body therein, at least one of the filaments being changeable in length at the part forming the space , so that the loop formed by the filament and the branch part changes in size.
(2) A wire for removing an intravascular foreign body as defined in (1) above, in which the branch part is constructed of a hollow wire, through which at least one of the filaments passes.
(3) A wire for removing an intravascular foreign body as defined in (1) or (2) above, in which the space forming part is designed such that its both ends are movable relative to the branch part.
(4) A wire for removing an intravascular foreign body as defined in (1) or (2) above, in which the space forming part has its one end fixed to one of the branch parts and its another end movably attached to another of the branch parts.
(5) A wire for removing an intravascular foreign body as defined in any of (1) to (4) above, which has means for controlling the minimum size of the loop.
(6) A wire for removing an intravascular foreign body as defined in any of (1) to (5) above, which has in the space forming part a plurality of projections projecting into the space for capturing the foreign body therein.
(7) A wire for removing an intravascular foreign body as defined in any of (1) to (6) above, which has in the space forming part a plurality of flexible thin fiber projecting into the space for capturing the foreign body therein.
(8) A wire for removing an intravascular foreign body as defined in any of (1) to (7) above, which has in the space forming part a plurality of projections projecting into the space for capturing the foreign body therein and a plurality of thin fiber (softer than the projections) projecting into the space therein.
(9) A medical instrument which includes the wire for removing an intravascular foreign body as defined in any of (1) to (8) above and a catheter having a lumen for receiving therein the wire for removing an intravascular foreign body.

According to the present invention, the object of removing an intravascular foreign body is achieved surely by changing the size of the loop formed with the filament part and the branch part.

In the embodiment in which the space forming part is provided with a plurality of projections or fibers, it is possible to surely prevent the foreign body slipping off from the space in which the foreign body is captured. In this way it is possible to capture and remove the foreign body.

### BRIEF DESCRIPTION OF THE DRAWINGS

Fig. 1 is a perspective view showing the wire for removing an intravascular foreign body pertaining to the first embodiment of the present invention.
Fig. 2 is a plan view (with a partial longitudinal sectional view) showing the wire for removing an intravascular foreign body, which is shown in Fig. 1.
Fig. 3 is a plan view (with a partial longitudinal sectional view) showing the wire for removing an intravascular foreign body, which is shown in Fig. 1.
Fig. 4 is a side view showing the wire for removing an intravascular foreign body, which is shown in Fig. 1.
Fig. 5 is a diagram illustrating how to use the wire for removing an intravascular foreign body which is shown in Fig. 1.
Fig. 6 is a diagram illustrating how to use the wire for removing an intravascular foreign body which is shown in Fig. 1.
Fig. 7 is a diagram illustrating how to use the wire for removing an intravascular foreign body which is shown in Fig. 1.
Fig. 8 is a diagram illustrating how to use the wire for removing an intravascular foreign body which is shown in Fig. 1.
Fig. 9 is a diagram illustrating how to use the wire for removing an intravascular foreign body which is shown in Fig. 1.
Fig. 10 is a plan view (with a partial longitudinal sectional view) showing the wire for removing an intravascular foreign body pertaining to the second embodiment of the present invention.
Fig. 11 is a plan view (with a partial longitudinal sectional view) showing the wire for removing an intravascular foreign body pertaining to the second embodiment of the present invention.

### DETAILED DESCRIPTION OF THE PREFERRED EMBODIMENTS

A detailed description is given below of the wire for removing an intravascular foreign body and the medical instrument of the present invention with reference to the preferred embodiments shown in the accompanying drawings.

### First Embodiment

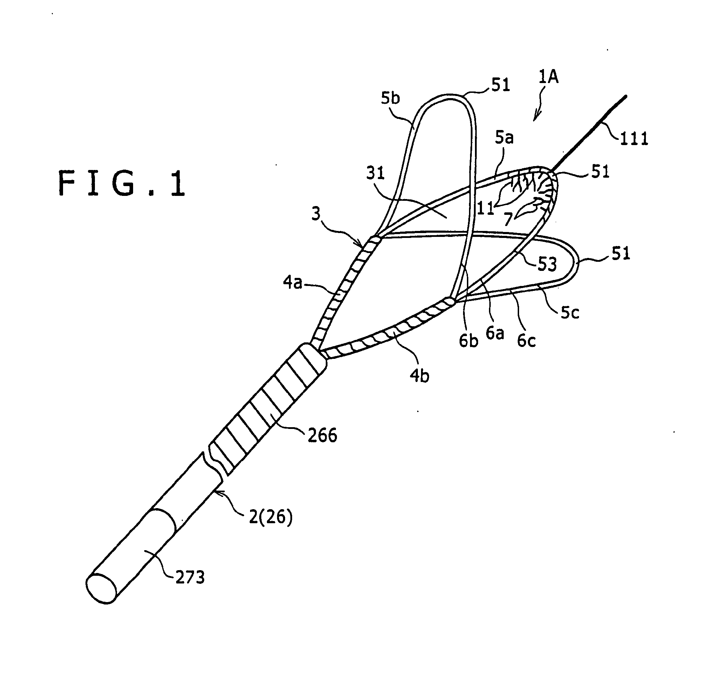

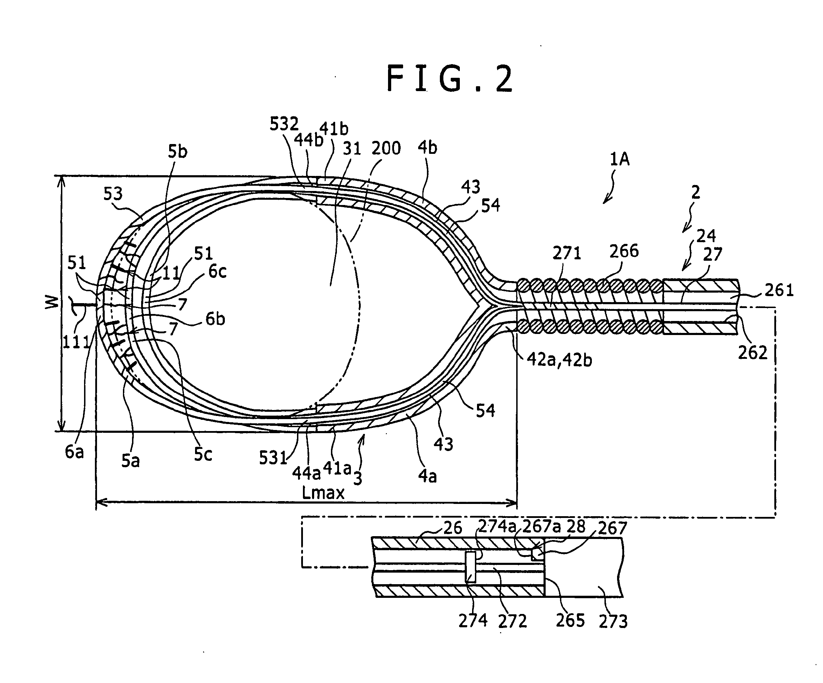

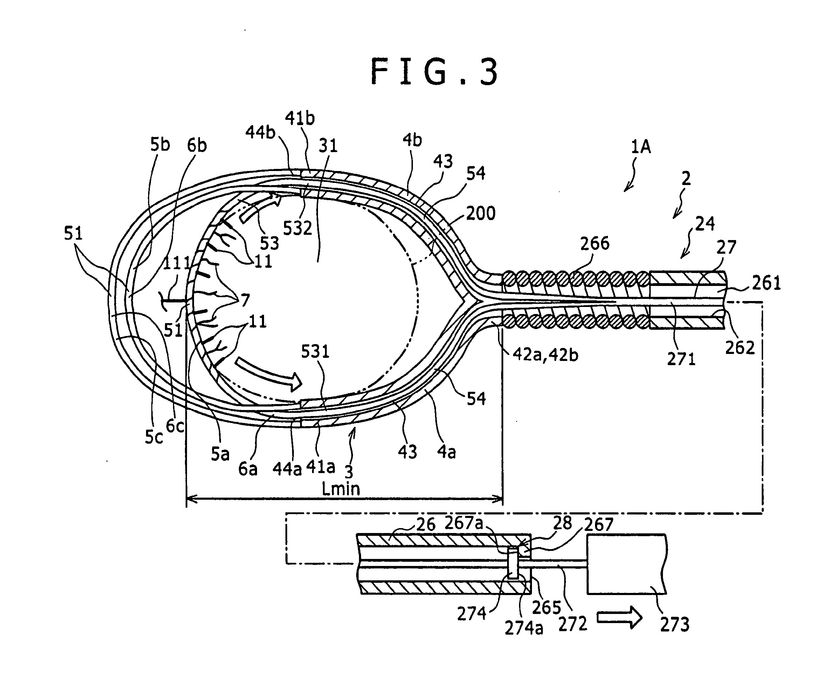

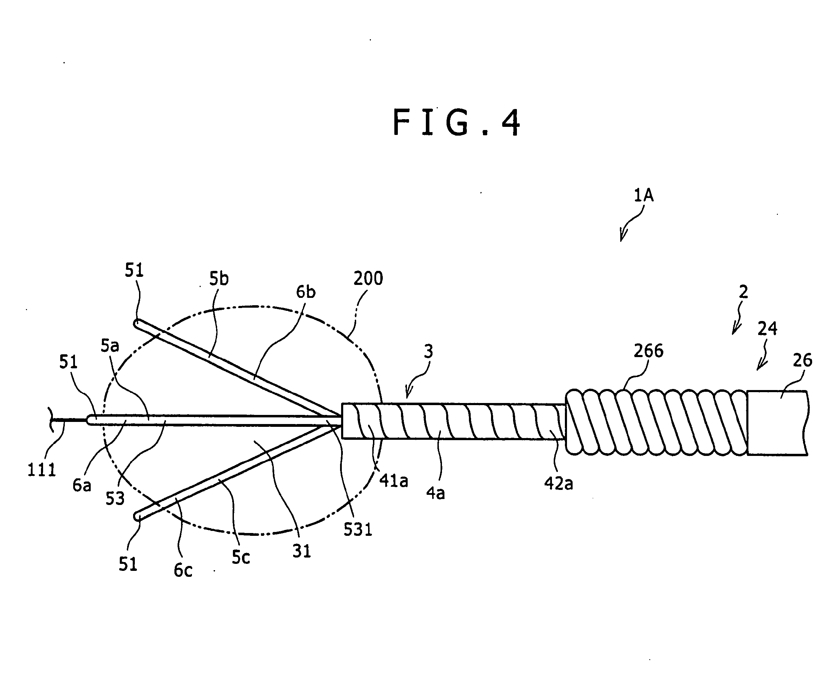

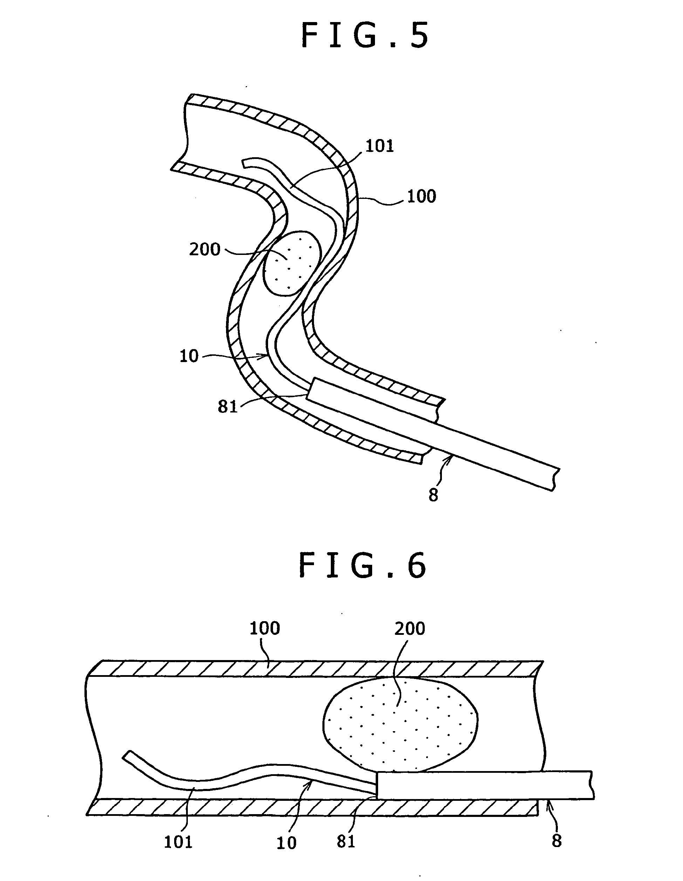

Fig. 1 is a perspective view showing the wire for removing an intravascular foreign body pertaining to the first embodiment of the present invention. Figs. 2 and 3 are plan views (with a partial longitudinal sectional view) showing the wire for removing an intravascular foreign body, which is shown in Fig. 1. Fig. 4 is a side view showing the wire for removing an intravascular foreign body, which is shown in Fig. 1. Figs. 5 to 9 are diagrams each illustrating how to use the wire for removing an intravascular foreign body which is shown in Fig. 1.

Incidentally, the terms "base end" and "forward end" in the following description are defined as follows. In Fig. 1, the upper right side is "forward end (distal end)" and the lower left side is "base end (proximal end)", and in Figs. 2 to 9, the left side is "forward end (distal end)" and the right side is "base end (proximal end)".

Fig. 1 shows the wire 1A for removing an intravascular foreign body, which is intended to capture and remove a foreign body (such as thrombus and clot) which causes embolism in the vessel. A foreign body will be referred to as "embolus 200" hereinafter.

The wire 1A for removing an intravascular foreign body is included a long wire body 2 and a capturing part 3 attached to the forward end of the wire body 2. The capturing part 3 is so designed as to capture the embolus 200 in the vessel 100. Each part is constructed as described in the following.

The wire body 2 shown in Fig. 2 includes a tube 26 and a linear body 27 which passes through the inside 261 of the tube 26. The wire body 2 has adequate rigidity and resilience (flexibility) over its entire length.

The tube 26 has a coil 266 arranged in (connected to) the forward end thereof. The coil 266 makes the forward end of the tube 26 (including the coil 266) more resilient (or flexible).

As shown in Figs. 2 and 3, the linear body 27 is constructed such that it can move relative to the tube 26. The linear body 27 is also constructed such that its base 272 can project from the base-end opening 265 of the tube 26 (See Fig. 3).

The linear body 27 has a manipulating member 273 attached to its base end 272, which is designed to manipulate (move) the linear body 27 in its lengthwise direction. The manipulating member 273 helps the operator grasp the linear body 27 during its operation and facilitates the operation of the linear body 27.

The parts constructing the wire body 2 may be formed from any materials without specific restrictions, such as metallic and plastic materials, which may be used alone or in combination.

The wire body 2 may vary in length depending on the position and size of the vessel 100 to which it is applied. A preferred length ranges from 500 to 4000 mm usually, and more preferred length ranges from 1500 to 2200 mm.

The wire body 2 (or the tube 26) may also vary in thickness (outside diameter) depending on the position and size of the vessel 100 to which it is applied. A preferred outside diameter is usually 0.1 to 2.0 mm on average, and more preferred length ranges from 0.25 to 0.9 mm.

If the wire 1A for removing an intravascular foreign body is to be inserted into a microcatheter, the outside diameter of the wire body 2 should preferably be 0.1 to 0.53 mm (0.004 to 0.021 inches), more preferably 0.25 to 0.46 mm (0.010 to 0.018 inches).

The microcatheter denotes a catheter for intravascular treatment and/or diagnosis which has an outside diameter no larger than 4 French (equal to approximately 1.33 mm). It is clearly distinguished from angiography catheters and guiding catheters having an outside diameter equal to or larger than 4 French.

The wire body 2 (or the tube 26) should preferably be composed of a first part (which is comparatively hard and is placed at the base end), a third part (which is comparatively soft and is placed at the forward end), and a second part (which is variable in flexibility and is placed at the intermediate position between the first part and the third part). In other words, the wire body 2 should preferably be formed in such a way that it gradually decreases in rigidity (flexural and torsional rigidity) in going from its base end to its forward end. The gradually changing rigidity permits the manipulation by hands to be certainly transmitted to the forward end 24 (including the coil 266) of the wire body 2. With such properties (that make the forward end 24 flexible), the wire body 2 easily proceeds and bends in the vessel 100 without damaging the vessel 100. Such properties permit the wire body 2 to transmit its twisting motion and its pushing motion while preventing kinking (or flexing). This contributes to higher safety.

The wire body 2 (or the tube 26) may have a coating layer on its outer surface for reduction of friction with the inside of the catheter 8 (mentioned later). The coating layer permits smooth insertion into and removal from the catheter. The coating layer may be formed from a fluorocarbon resin (such as polytetrafluoroethylene or "Teflon (registered trademark)") or a hydrophilic polymer which becomes lubricious in a wet condition.

The linear body 27 may also have a coating layer on its outer surface (as in the case of the tube 26) for reduction of friction with the internal surface 262 of the tube 26. The coating layer produces the same effect as mentioned above.

At the forward end of the wire body 2 is the capturing part 3, which is open in its natural state as shown in Figs. 2 and 3. "Open" means that the width expands in the vertical direction in Figs. 2 and 3. This state is referred to "expanded state" hereinafter. In its expanded state, the capturing part 3 forms therein the space for capturing therein the foreign body or the embolus 200 in the vessel 100.

The capturing part 3 is changeable in shape or collapsible. In other words, it becomes small in size so that it is received (held) in the catheter 8. This state is referred to as "collapsed state" hereinafter.

The capturing part 3 is capable of changing from its collapsed state to its expanded state by its own elasticity.

In the following description, it is assumed that the capturing part 3 has its shape and size in its expanded state (or natural state), unless otherwise mentioned.

As shown in Fig. 1, the capturing part 3 is included two branch wires (branch part) 4a and 4b and three filaments 5a, 5b, and 5c. The two branch wires branch (and extend apart) from the forward end of the coil 266 (or the forward end of the wire body 2). The three filaments extend from the branch wire 4a to the branch wire 4b.

The branch wires 4a and 4b are constructed of hollow wires. The hollow wires include not only pipe-like wires but also coils and braided wires with a hollow inside. The example shown is constructed as follows. The coil 266 is composed of at least four fine wires. These four wires are separated into two wires at the forward end of the coil 266. These two wires are so twisted as to make the hollow twisted wires (which are the branch wires 4a and 4b). It is also possible to form the branch wires 4a and 4b by fixing a branched pipe-like member to the forward end of the coil 266.

The base ends 42a and 42b of the branch wires 4a and 4b (loop wires 6a, 6b and 6c) are fixed to the forward end of the coil 266, so that the hollow part 43 of the branch wires 4a and 4b communicates with the bore 261 of the tube 26.

No specific restriction is imposed on the method of fixing the branch wires 4a and 4b to the coil 266. One method may consist of winding the base end 42a and 42b of the branch wires 4a and 4b around the forward end of the coil 266 and then performing brazing, welding, or adhesion with an adhesive.

The three filaments 5a, 5b, and 5c extend from the forward end 41a of the branch wire 4a to the forward end 41b of the branch wire 4b.

As shown in Fig. 2, the filament 5a has the connecting part 54 which connects the space forming part 53 (which forms the space 31 in which is captured the foreign body) to the linear body 27 of the wire body 2.

The space forming part 53 projects forward from the forward opening 44a and 44b of the branch wire 4a and 4b. The space forming part 53 assumes an arch-like shape which curves in the same direction.

The connecting part 54 is formed at the ends 531 and 532 of the space forming part 53 respectively. Eachof the connecting part 54 passes through the bore 43 of the branch wires 4a and 4b and is fixed to the forward end 271 of the linear body 27. No specific restriction is imposed on the method of fixing. The method mentioned above for the branch wires 4a and 4b may be used.

The connecting part 54 permits the space forming part 53 to be introduced easily into the hollow part 43 of the branch wires 4a and 4b.

As shown in Fig. 4, the filament 5a constructed as mentioned above is positioned approximately on the plane (perpendicular to the paper of Fig. 4) passing through the extension line of the central axis of the wire body 2. In other words, the filament 5a overlaps approximately with the extension line of the central axis of the wire body 2 in the side view (Fig. 4).

As shown in Fig. 2, the filaments 5b and 5c curve in such a way that their apex 51 bulges toward the forward end, and they connect the forward end 41a of the branch wire 4a to the forward end 41b of the branch wire 4b.

Also, the filaments 5b and 5c are inclined such that the distance from the extension line of the central axis of the wire body 2 increases in going in the direction toward the forward end. In other words, in Fig. 4 (side view), the filament 5b is inclined left-upward and the filament 5c is inclined left-downward.

The filaments 5b and 5c fixed in this manner produce the following effect. When the capturing part 3 captures the embolus 200, it keeps the captured embolus 200 from being carried by blood flow.

The capturing part 3 has the capturing space 31 (for capturing or trapping the embolus 200 therein) which is surrounded by the branch wires 4a and 4b and the filaments 5a, 5b, and 5c. In other words, the filaments 5a, 5b, and 5c form a basket-like part for holding the foreign body.

The wire 1A for removing an intravascular foreign body has three loop wires 6a, 6b, and 6c which extend from the forward end of the wire body 2. The loop wires are constructed from the branch wires 4a and 4b and the filaments 5a, 5b, and 5c. Each of the loop wires 6a, 6b, and 6c extends from the forward end of the wire body 2 and returns to the forward end of the wire proper 2 after forming a loop.

The wire 1A for removing an intravascular foreign body, which is constructed as mentioned above, has the manipulating member 273 which can be moved toward the base end, as mentioned above. In this way it is possible to change the size of the loop wire 6a. Thus, the loop wire 6a can vary in size from the maximum (the first state shown in Fig. 2) to the minimum (the second state shown in Fig. 3).

It is possible to make the loop wire 6a to change from the first state to the second state in the following manner.

First, the manipulating member 273 is pulled toward the base end, so that the ends 531 and 532 of the space forming part 53 are pulled (moved) into the hollow part 43 of the branch wires 4a and 4b. As the result, the projecting length of the space forming part 53 changes (or becomes short). That is, the loop wire 6a changes from the first state into the second state.

The embolus 200 which has been captured in the foreign body capturing space 31 when the loop wire 6a is in the first state is tightened (and held) by the loop wire 6a as the loop wire 6a changes into the second state. See Figs. 2 and 3. In this way it is possible to prevent the captured embolus 200 from slipping off from the foreign body capturing space 31 and hence it is possible to surely capture the embolus 200 and eventually remove the embolus 200 from the vessel 100.

The wire 1A for removing an intravascular foreign body which has been mentioned above has the space forming part which changes in length by one filament. This construction may be modified such that the space forming part changes in length by two or more filaments.

It is desirable to form a coating layer (like the one explained above for the tube 26 of the wire proper 2) on the outer surface of the connecting part 54 of the filament 5a and the linear body 27 of the wire body 2,it is also desirable to form a coating layer (like the one explained above for the tube 26 of the wire body 2) on the inner surface of the branch wires 4a and 4b and inner surface (internal peripheral surface 262) of the tube 26 of the wire body 2.

As shown in Fig. 3, the control means 28 is intended to control the size of the loop wire 6a in the second state. This size is referred to as "minimum loop diameter" hereinafter.

The control means 28 is included a projecting part 267 and an expanded part 274. The projecting part 267 projects in the radial direction from the inside 262 in the neighborhood of the base opening 265 of the tube 26. The expanded part 274 is a part which expands in the radial direction from the outside of the linear body 27 in the bore 261 of the tube 26.

The expanded part 274 is so formed as to fix a ring-shaped member to the linear body 27.

The projecting part 267 of the tube 26 and the expanded part 274 of the linear body 27 are constructed such that the face 267a at the forward end side of the projecting part 267 comes into contact with the face 274a at the base end side of the expanded part 274 when the loop wire 6a assumes the second state (or when the linear body 27 moves toward the base end relative to the tube 26). This prevents the linear body 27 from moving excessively toward the base end.

The control means 28 constructed as mentioned above protects the embolus 200 from being excessively tightened by the loop wire 6a. In this way it is possible to prevent the embolus 200 from being broken by the loop wire 6a.

Incidentally, the size of the minimum loop diameter (the length indicated by Lₘᵢₙ in Fig. 3) is not specifically restricted so long as it is smaller than Lₘₐₓ. If Lₘₐₓ is 8 to 15 mm, Lₘᵢₙ should preferably be 1 to 7.5 mm, more preferably 3 to 5 mm.

The expanded part 274 is capable of adjusting its position relative to the linear body 27. In this case, the minimum loop diameter is adjustable by this adjustment. In this way it is possible to establish the minimum loop diameter according to the size of the embolus 200.

The outside diameter of the branch wire 4a and 4b are not specifically restricted. It should preferably be 0.05 to 0.9 mm, more preferably 0.1 to 0.5 mm.

Also, the outside diameter of the filaments 5a, 5b, and 5c are not specifically restricted; it should preferably 0.025 to 0.2 mm, more preferably 0.05 to 0.1 mm.

According to the present invention, the size of the capturing part 3 may be established as desired. It varies depending on the vessel thickness and the case of disease. The overall length indicated by Lₘₐₓ in Fig. 2 is usually 2 to 40 mm, preferably 4 to 20 mm. The outside diameter or width (indicated by W in Fig. 2) of the capturing part 3 in its expanded state should preferably be 1 to 30 mm, more preferably 2 to 5 mm.

For use in the cerebral artery end (M1 portion), the capturing part 3 should have an overall length (indicated by Lₘₐₓ in Fig. 2) of at least 7 mm, preferably 8 to 15 mm, and an outside diameter (indicated by W in Fig. 2) of 2 to 5 mm, in its expanded state. This is because the inside diameter of the vessel in the cerebral artery end (M1 portion), where brain infarction occurs frequently, is about 3 to 4 mm and, according to doctors' experience, the size of thrombus causing embolus is about 3 mm in outside diameter and about 7 mm in length in many cases.

As mentioned above, the capturing part 3 is capable of deforming into the collapsed state so that it can pass through (or can be inserted into) the lumen 82 of the catheter 8. According to the present invention, the capturing part 3 is comparatively simple in structure, so that it can be made thin easily in its collapsed state.

According to the present invention, the capturing part 3 returns to its expanded state from its collapsed state by its own resilience owing to the comparatively highly rigid (comparatively thick) branch wires 4a and 4b. Moreover, the comparatively flexible (comparatively thin) filaments 5a, 5b, and 5c surely hold the embolus 200, while preventing it from slipping off. Thus the capturing part 3 can be small in diameter (in its collapsed state) when it captures the embolus 200.

The capturing part 3 can be as small in diameter as the ordinary guide wire; therefore, the wire 1A for removing an intravascular foreign body can be used in combination with the existing microcatheter.

The ratio of W/W' (where W is the outside diameter (W mentioned above) of the capturing part 3 in its expanded state and W' is the outside diameter (width) of the capturing part 3 in its collapsed state) should preferably be about 1.1 to 20, more preferably 1.1 to 10.

In the case of use in combination with a microcatheter, the outside diameter W' in the collapsed state should be smaller than 0.53 mm (0.021 inches), more preferably smaller than 0.46 mm (0.018 inches).

The capturing part 3 returns from its collapsed state to its expanded state by its own elasticity as the wire 1A for removing an intravascular foreign body (the wire body 2) is advanced relative to the catheter 8 so that the capturing part 3 is pushed out (exposed) from the forward opening 81 of the catheter 8.

As the wire 1A for removing an intravascular foreign body is retreated toward the base end of the catheter 8, the base end of the branch wires 4a and 4b come into contact with the edge of the forward opening 81 and then enters the catheter 8. Thus the distance between the branch wires 4a and 4b decreases.

As the wire 1A for removing an intravascular foreign body is retreated further toward the base end, the capturing part 3 assumes the collapsed state automatically, with the distance between the branch wires 4a and 4b decreased, and it is received (held) again in the catheter 8.

As mentioned above, the capturing part 3 deforms from its expanded state to its collapsed state automatically and vice versa as it enters or leaves the forward opening 81 of the catheter 8.

The capturing part 3 (loop wires 6a, 6b, 6c) should preferably be formed from any radiopaque material which is not specifically restricted. It includes, for example, gold, platinum, platinum-iridium alloy, tungsten, tantalum, palladium, lead, silver, and their alloys and compounds. Such radiopaque materials facilitate manipulation (to capture the embolus 200 by the capturing part 3) with the help of X-ray radioscopy and the like.

The capturing part 3 (loop wires 6a, 6b, 6c) may be formed from various metallic materials and plastic (mono- or multi-) filaments. Metallic materials include stainless steel (such as SUS 304), β-titanium steel, Co-Cr alloy, piano wire, platinum-iridium alloy (Pt₉₀-Ir₁₀, Pt₈₀-Ir₂₀), noble metal alloy, nickel-titanium alloy, and other alloys having springy properties.

Of these metallic materials for the capturing part 3, the one which exhibits superelasticity in the living organism is preferable. It surely changes from the collapsed state to the expanded state, and vice versa.

The alloy that exhibits superelasticity in the living organism denotes the one which restores nearly to its original shape after deformation (bending, stretching, and compressing) to an extent at which ordinary metals undergo plastic deformation at the living body temperature (about 37°C). It includes so-called shape memory alloy and superelastic alloy.

The shape memory alloy and superelastic alloy are not specifically restricted. Titanium alloys (Ti-Ni, Ti-Pd, Ti-Nb-Sn) and copper alloys are preferable. The titanium alloys should preferably be composed of 30 to 52 atom% of titanium, with the remainder being nickel and one or more optional elements (equal to or less than 10 atom%). The optional elements, which are not specifically restricted, may be selected from iron, cobalt, platinum, palladium, and chromium (each equal to or less than 3 atom%), and copper and vanadium (each equal to or less than 10 atom%).

Preferred superelastic alloys are those which undergo transformation from austenite phase at normal temperature or body temperature (about 37°C) into martensite phase under stress.

The surface of the capturing part 3 should be provided with means for preventing the embolus 200 (which has been captured) from slipping off from the capturing part 3. Such anti-slipping means increases friction between the capturing part 3 and the embolus 200, thereby allowing the capturing part 3 to surely hold (capture) the embolus 200.

The anti-slipping means is not specifically restricted. It may be formed by coating with an elastic material (such as rubber) having a comparatively high coefficient of friction or by sand blasting which produces fine rough surfaces or surface irregularities.

The outer surface of the capturing part 3 (filaments 5a, 5b, 5c) may be provided with a coating layer as explained above for the wire body 2. The coating layer permits the capturing part 3 to be inserted into and removed from the catheter 8 easily.

The filament 5a and the liner body 27 of the wire body 2 may be constructed of separate materials or may be integrally formed with the same material.

As shown in Fig. 2, the filament 5a (the space forming part 53) has a plurality of projections 11 which project into the foreign body capturing space 31.

The projections 11 may be formed in any manner which is not specifically restricted. One method may consist of winding one end of a flexible linear body (wire) around the filament 5a, while allowing the other small end to project.

Other ends of each linear body are gathered (or twisted together) in the vicinity of the apex 51 of the filament 5a so that they project into the forward end. Thus other ends of the linear bodies function as the guide wire 111.

Incidentally, each projection 11 may be formed from any material which is not specifically restricted. Metallic materials or plastic materials may be used alone or in combination with one another.

The length (on average) of each projection is not specifically restricted; it should preferably be 0.1 to 5 mm, more preferably 0.5 to 2 mm.

As shown in Fig. 2, the filament 5a (the space forming part 53) has a plurality of flexible fine fibers 7 projecting into the foreign body capturing space 31. Each fine fiber 7 should preferably be softer than the projection 11.

Each fine fiber 7 may be formed in any manner which is not specifically restricted. For example, one method may consist of winding a fibrous body (having fine fibers 7) around the filament 5a or attaching fine fibers to the filament 5b by static flocking.

The fine fibers 7 may be formed from any material which is not specifically restricted. The material includes radiation-transparent fibers such as Dacron (polyester), polyglycolic acid, polylactic acid, fluoropolymer (polytetrafluoroethylene), nylon (polyamide), cotton, and silk. The material also includes metallic yarn coated with radiation-transparent fiber or radiation-opaque fiber.

The length (on average) of each fine fiber 7 is not specifically restricted; it should preferably be 0.1 to 5 mm, more preferably 0.5 to 3 mm.

The projection 11 and the fine fibers 7 formed as mentioned above make it possible to surely capture the embolus 200. In the case of a comparatively hard embolus 200, the projection 11 stabs the embolus 200, thereby preventing it from slipping off from the foreign body capturing space 31. In the case of a comparative soft embolus 200, the fine fibers 7 hang on to the embolus 200, thereby preventing it from slipping off from the foreign body capturing space 31.

Incidentally, the projections 11 may be formed not only on the filament 5a but also on the filaments 5b and 5c or on any one of the filaments 5a, 5b, and 5c.

The fine fiber 7 may be formed not only on the filament 5a but also on the filaments 5b and 5c or on any one of the filaments 5a, 5b, and 5c.

The medical instrument 9 according to the present invention is included the wire 1A for removing an intravascular foreign body and the catheter 8.

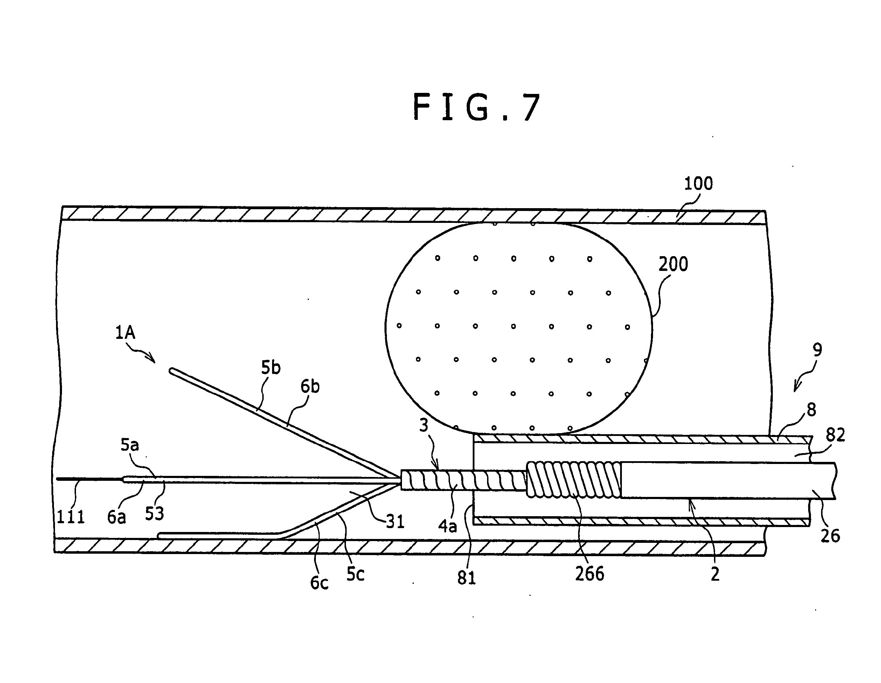

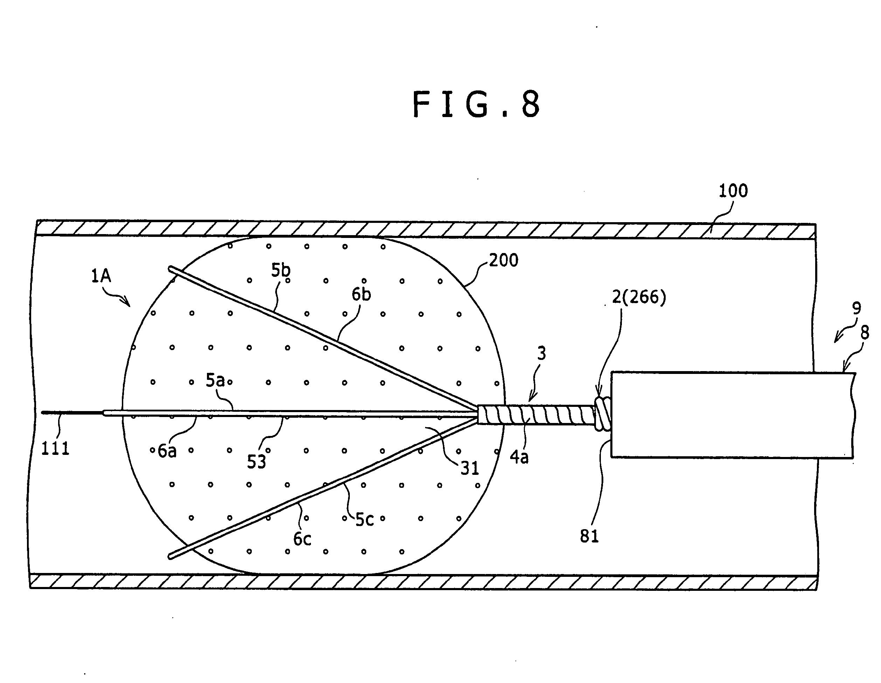

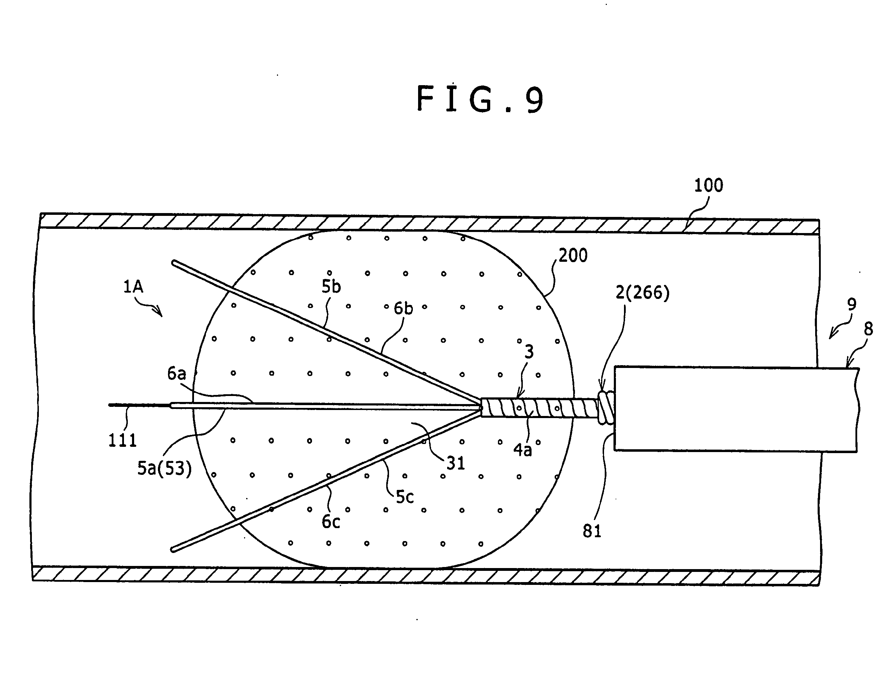

A detailed description is given below of one way of using the wire 1A for removing an intravascular foreign body.
[1] Fig. 5 depicts the vessel 100 which is clogged with the embolus 200 (such as thrombus) which hinders blood flow. The embolus 200 is almost immobile because it is pushed against the inner wall of the vessel 100 by blood pressure.
   The first step is to insert the catheter (microcatheter) 8 and the guide wire 10 (which has been passed through the lumen 82 of the catheter 8) into the vessel 100. The second step is to project a forward end 101 of the guide wire 10 from the forward open end 81 of the catheter 8 beyond the embolus 200. In other words, the second step is carried out such that the forward end 101 of the guide wire 10 passes through the gap between the embolus 200 and the inner wall of the vessel 100 and gets over the embolus 200. This operation can be easily accomplished by using the guide wire 10 (micro-guide wire) which has good lubricity.
[2] After the forward end 101 of the guide wire 10 has got over the embolus 200, the catheter 8 is advanced relative to the guide wire 10, so that the forward end of the catheter 8 gets in the gap between the embolus 200 and the inner wall of the vessel 100, as shown in Fig. 6. This operation is easy because the forward end of the catheter 8 smoothly gets in the gap along the guide wire 10.
   According to the conventional therapy, the above-mentioned stage is followed by injection of a thrombolitic agent through the catheter 8. However, it is often experienced by doctors that there are emboli 200 which are not dissolved by a thrombolitic agent or dissolution by a thrombolitic agent takes a long time. The present invention is effective in such a case.
[3] The step shown in Fig. 6 is followed by the next step in which the guide wire 10 is removed and the wire 1A for removing an intravascular foreign body is inserted into the lumen 82 of the catheter 8. As shown in Fig. 7, the capturing part 3 is caused to project from the forward opening 81 of the catheter 8, so that the capturing part 3 (which has been in its collapsed state) expands automatically by its own elasticity and turned into its expanded state. When the capturing part assumes its expanded state, it forms the foreign body capturing space 31 that captures the embolus 200.
[4] Then, the catheter 8 is slightly moved toward the base end and the forward end of the catheter 8 is retreated to the back end of the embolus 200. Now, the embolus 200 is captured (scooped) by the foreign body capturing space 31 of the capturing part 3, as shown in Fig. 8. In other words, the embolus 200 enters the foreign body capturing space 31 from the upper side shown in Figs. 7 and 8.
[5] The loop wire 6a is made small by operating the manipulating member 273 of the linear body 27, so that the embolus 200 is drawn to the branch wires 4a and 4b. The loop wire 6a is made smaller further, so that the embolus 200 is tightened by the loop wire 6a, as shown in Fig. 9.
[6] The wire 1A for removing an intravascular foreign body and the catheter 8 are removed all together, with the state (the tightened state) shown in Fig. 9 maintained. Thus, the embolus 200 is captured in the guiding catheter or sheath introducer (not shown).

### Second Embodiment

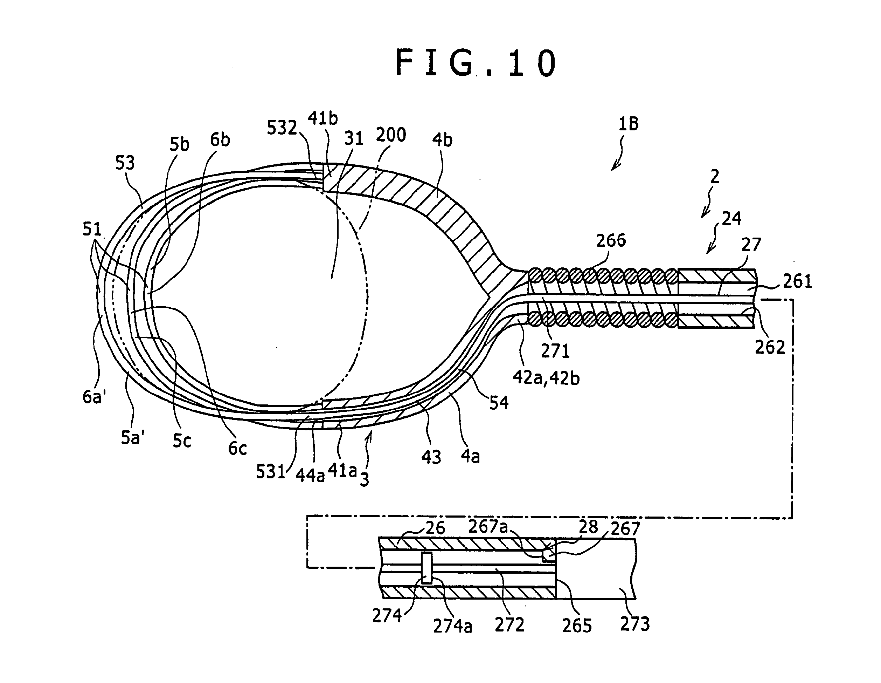

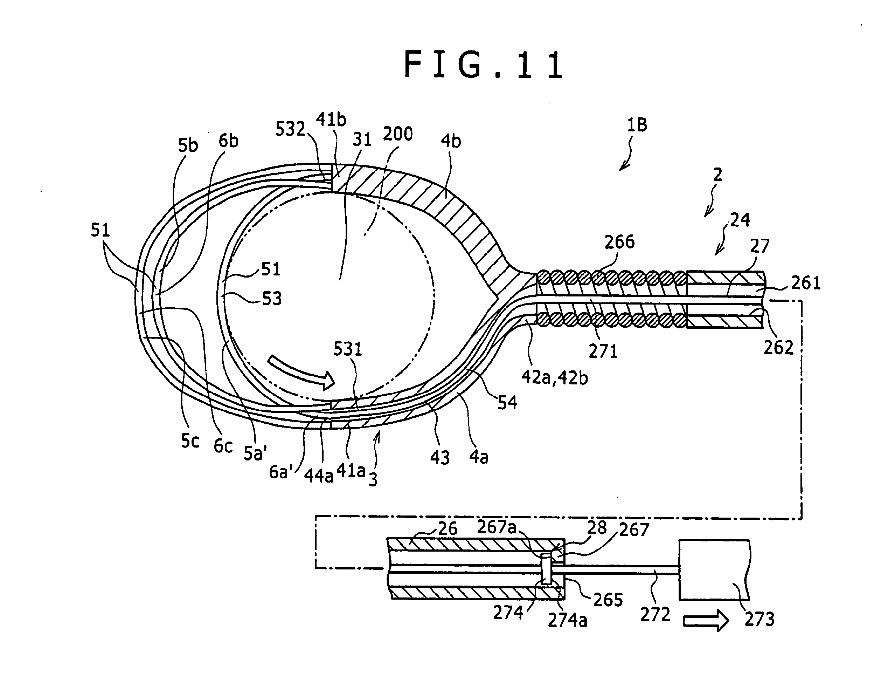

Figs. 10 and 11 are plan views (with a partial longitudinal sectional view) showing the wire for removing an intravascular foreign body pertaining to the second embodiment of the present invention.

These figures will be referenced in the following description about the wire for removing an intravascular foreign body pertaining to the second embodiment of the present invention. Stress is placed on differences between the first embodiment and the second embodiment, and description of those items common to the two embodiments is omitted.

The wire for removing an intravascular foreign body pertaining to the second embodiment is identical with that pertaining to the first embodiment except for the construction of the branch wire and the filaments.

As shown in Fig. 10, the wire 1B for removing an intravascular foreign body has the branch wire 4b which is a solid wire.

The filament 5a' (the space forming part 53) has its end 532 fixed to the forward end 41b of the branch wire 4b. Fixing may be accomplished in any manner without specific restrictions. One way is to wind the end 532 of the filament 5a around the forward end 41b of the branch wire 4b and bond them together by brazing, welding, or adhesion with an adhesive.

The another end 531 of the filament 5a' (the space forming part 53) is movably attached to the branch wire 4a, as the same way as that of the first embodiment.

The wire 1B for removing an intravascular foreign body constructed as mentioned above permits the loop wire 6a' (which is constructed from the branch wires 4a and 4b and the filament 5a') to change in size as the manipulating member 273 is moved toward the base end. Because of this change, the loop wire 6a' assumes the first state (Fig. 10) with its maximum size and the second state (Fig. 11) with its minimum size.

When the loop wire 6a' is in its first state, the embolus 200 is captured in the foreign body capturing space 31. The captured embolus 200 is tightened by the loop wire 6a' when the loop wire 6a' assumes its second state. See Figs. 10 and 11. In this way it is possible to prevent the captured embolus 200 from slipping off from the foreign body capturing space 31 and hence to surely capture the embolus 200 and eventually to remove the embolus 200 from the vessel 100.

In the case where the filament 5a' is provided with the projection 11 and the fine fiber 7, the projection 11 and the fine fiber 7 turns as the loop wire 6a' shrinks or turns in the direction of arrow (or counterclockwise) as shown in Fig. 11. The turning of the projection 11 and the fine fiber 7 facilitate the rotation of the embolus 200 captured by the projection 11 and the fine fiber 7. If it is not desirable that the embolus 200 rotates, the filament 5a' should not be provided with the projection 11 and the fine fiber 7.

The filaments 5b and 5c may also be provided with the projection 11 and the fine fiber 7, although not shown in the figure.

The space forming part 53 varies in the rate of reduction of its length depending on embodiments as is apparent from the structure of the loop wire 6a in the first embodiment and the structure of the loop wire 6a' in the second embodiment.

For example, in the first embodiment, the loop wire 6a decreases in size (periphery) by 2 mm when the manipulating part 273 is moved by 1 mm.

Also, in the second embodiment, the loop wire 6a' decreases in size by 1 mm when the manipulating part 273 is moved by 1 mm.

The fact that the space forming part 53 varies in the rate of reduction of its length depending on embodiments (wire 1A and 1B) produces the following effect. If it is desirable to shrink the loop wire rapidly, it is recommended to use the wire 1A. If it is desirable to shrink the loop wire slowly, it is recommended to use the wire 1B.

Selection of the wires 1A and 1B should be made as follows. Where a subtle adjustment is necessary for the size of the loop wire, it is recommended to use the wire 1B, because the diameter of the forward end 271 is smaller than that of the wire 1A. This means that the loop wire can be made smaller with less friction during operation.

The foregoing is about the wire for removing an intravascular foreign body and the medical instrument according to the present invention. It is not intended to restrict the scope of the present invention. The constituents of the wire and medical instrument may be modified or replaced by (or reinforced with) those which function in the same way.

Two or more of the features in the above-mentioned embodiments may be combined to complete the wire for removing an intravascular foreign body.

For example, the first embodiment may be modified such that the filaments at both sides are constructed so that the length of the space forming part changes as in the case of the central filament. This modification permits the same wire to vary in the rate of reduction of the length of the space forming part differently for the central filament and the outside filaments. The effect of this modification is that the loop wire can be reduced in size in conformity with the shape of the embolus. This ensures the capture of the foreign body.

In this case, the embolus is tightened in three directions, so that it is possible to capture (hold) the embolus more certainly.

The first embodiment may be modified such that both ends of the space forming part are fixed to different linear bodies instead of being fixed to one linear body. The number of the branch wires is not limited to two; it may be three or more.

The number of filaments is not limited to three; it may be two or four or more.

The number of filaments for the space forming part which varies in length is not limited to one; it may be two or more.

The wire for removing an intravascular foreign body includes a flexible wire body, at least two branch parts branching out from the forward end of the wire body, and a plurality of filaments extending across the branch part. The branch parts and the filaments form a space for capturing a foreign body therein, at least one of the filaments being changeable in length at the part forming the space for capturing a foreign body therein, so that the loop formed by the filament and the branch part changes in size. The wire is capable of surely capturing and removing a foreign body in the vessel.

## Claims

1. A wire for removing an intravascular foreign body, comprising: a flexible wire body (2), at least two branch parts (4a, 4b) branching out from the forward end of said wire body, and a plurality of filaments (5a, 5b, 5c) extending across said branch part, said branch parts and said filaments forming a space for capturing a foreign body (200) therein, at least one of said filaments (5a) being changeable in length at the part (53) forming the space, so that the loop formed by said filament and said branch part changes in size;
**characterized in that**
at least one filament (5a) forming the space forming part (53), having both ends (531, 532) movable, and at least one filament (5b, 5c) having both ends non-movable.

2. The wire for removing an intravascular foreign body as defined in Claim 1, in which the branch part (4a, 4b) is constructed of a hollow wire, through which at least one of the filaments (5a, 5b, 5c) passes.

3. The wire for removing an intravascular foreign body as defined in Claim 1 or 2, in which the space forming part (53) is designed such that its both ends are movable relative to the branch part (4a, 4b).

4. The wire for removing an intravascular foreign body as defined in Claim 1 or 2, in which the space forming part (53) has its one end fixed to one of the branch parts (4a) and its another end movably attached to another of the branch parts (4b).

5. The wire for removing an intravascular foreign body as defined in any of Claims 1 to 4, which has means (28) for controlling the minimum size of the loop.

6. The wire for removing an intravascular foreign body as defined in any of Claims 1 to 5, which has in the space forming part (53) a plurality of projections (11) projecting into the space.

7. The wire for removing an intravascular foreign body as defined in any of Claims 1 to 6, which has in the space forming part (53) a plurality of flexible thin fiber (7) projecting into the space.

8. The wire for removing an intravascular foreign body as defined in any of Claims 1 to 7, which has in the space forming part (53) a plurality of projections (11) projecting into the space and a plurality of thin fiber (7) softer than said projections (11) projecting into the space.

9. A medical instrument which comprises the wire for removing an intravascular foreign body as defined in any of Claims 1 to 8 and a catheter having a lumen for receiving the wire for removing an intravascular foreign body (200) therein.

## Patentansprüche

1. Draht zum Entfernen eines intravaskulären Fremdkörpers mit:
einem flexiblen Drahtkörper (2),
zumindest zwei Abzweigungsteilen (4a, 4b), die von dem vorderen Ende des Drahtkörpers heraus abzweigen, und
einer Vielzahl an Filamenten (5a, 5b, 5c), die sich über den Abzweigungsteil erstrecken, wobei die Abzweigungsteile und die Filamente einen Raum ausbilden, um in diesem einen Fremdkörper (200) einzufangen, wobei an zumindest einem der Filamente (5a) die Länge an dem Teil (53), der den Raum ausbildet, so änderbar ist, dass die durch das Filament und den Abzweigungsteil ausgebildete Schleife ihre Größe ändert;
**dadurch gekennzeichnet dass**
zumindest ein Filament (5a), das das den Raum ausbildenden Teil (53) ausbildet, zwei bewegliche Enden (531, 532) hat, und zumindest ein Filament (5b, 5c) zwei nicht bewegliche Enden hat.

2. Draht zum Entfernen eines intravaskulären Fremdkörpers gemäß Anspruch 1, wobei das Abzweigungsteil (4a, 4b) aus einem hohlen Draht aufgebaut ist, durch den zumindest eines der Filamente (5a, 5b, 5c) tritt.

3. Draht zum Entfernen eines intravaskulären Fremdkörpers gemäß Anspruch 1 oder 2, wobei der Raum ausbildende Teil (53) derart gestaltet ist, dass seine beiden Enden relativ zu dem Abzweigungsteil (4a, 4b) beweglich sind.

4. Draht zum Entfernen eines intravaskulären Fremdkörpers gemäß Anspruch 1 oder 2, wobei das den Raum ausbildende Teil (53) ein Ende hat, das an einem der Abzweigungsteile (4a) befestigt ist, und wobei sein anderes Ende beweglich an dem anderen der Abzweigungsteile (4b) angebracht ist.

5. Draht zum Entfernen eines intravaskulären Fremdkörpers gemäß einem der Ansprüche 1 bis 4, der eine Einrichtung (28) zum Steuern der minimalen Größe der Schleife hat.

6. Draht zum Entfernen eines intravaskulären Fremdkörpers gemäß einem der Ansprüche 1 bis 5, der in dem den Raum ausbildenden Teil (53) eine Vielzahl an Vorsprüngen (11) aufweist, die in den Raum hinein vorragen.

7. Draht zum Entfernen eines intravaskulären Fremdkörpers gemäß einem der Ansprüche 1 bis 6, der in dem den Raum ausbildenden Teil (53) eine Vielzahl an flexiblen dünnen Fasern (7) aufweist, die in den Raum hinein vorragen.

8. Draht zum Entfernen eines intravaskulären Fremdkörpers gemäß einem der Ansprüche 1 bis 7, der in dem den Raum ausbildenden Teil (53) eine Vielzahl an Vorsprüngen (11), die in den Raum hinein vorragen, und eine Vielzahl an dünnen Fasern (7) hat, die weicher als die Vorsprünge (11) sind, die in den Raum hinein vorragen.

9. Medizinisches Instrument mit
dem Draht zum Entfernen eines intravaskulären Fremdkörpers gemäß einem der Ansprüche 1 bis 8 und
einem Katheder mit einem Lumen zum Aufnehmen des Drahtes zum Entfernen eines intravaskulären Fremdkörpers (200).

## Revendications

1. Fil pour enlever un corps étranger intravasculaire, comprenant : un corps de fil flexible (2), au moins deux parties en ramification (4a, 4b) partant de l'extrémité avant dudit corps de fil, et une pluralité de filaments (5a, 5b, 5c) s'étendant au travers de ladite partie en ramification, lesdites parties en ramification et lesdits filaments formant un espace pour capturer un corps étranger (200) dans celui-ci, au moins un desdits filaments (5a) étant modifiable en longueur au niveau de la partie (53) formant l'espace, de façon à ce que la boucle formée par ledit filament et ladite partie en ramification change de taille ;
**caractérisé en ce que**
au moins un filament (5a) formant la partie (53) formant l'espace, ayant les deux extrémités (531, 532) mobiles, et au moins un filament (5b, 5c) ayant les deux extrémités non mobiles.

2. Fil pour enlever un corps étranger intravasculaire selon la revendication 1, dans lequel la partie en ramification (4a, 4b) est constituée d'un fil creux, à travers lequel au moins un des filaments (5a, 5b, 5c) passe.

3. Fil pour enlever un corps étranger intravasculaire selon la revendication 1 ou 2, dans lequel la partie (53) formant l'espace est conçue de façon à ce que ses deux extrémités soient mobiles par rapport à la partie en ramification (4a, 4b).

4. Fil pour enlever un corps étranger intravasculaire selon la revendication 1 ou 2, dans lequel la partie (53) formant l'espace a une extrémité fixée à l'une des parties en ramification (4a) et son autre extrémité attachée de façon mobile à une autre des parties en ramification (4b).

5. Fil pour enlever un corps étranger intravasculaire selon l'une quelconque des revendications 1 à 4, qui a un moyen (28) pour maîtriser la taille minimum de la boucle.

6. Fil pour enlever un corps étranger intravasculaire selon l'une quelconque des revendications 1 à 5, qui a dans la partie (53) formant l'espace une pluralité de projections (11) se projetant à l'intérieur de l'espace.

7. Fil pour enlever un corps étranger intravasculaire selon l'une quelconque des revendications 1 à 6, qui a dans la partie (53) formant l'espace une pluralité de fibres minces flexibles (7) se projetant à l'intérieur de l'espace.

8. Fil pour enlever un corps étranger intravasculaire selon l'une quelconque des revendications 1 à 7, qui a dans la partie (53) formant l'espace une pluralité de projections (11) se projetant à l'intérieur de l'espace et une pluralité de fibres minces (7) plus souples que lesdites projections (11) se projetant à l'intérieur de l'espace.

9. Instrument médical qui comprend le fil pour enlever un corps étranger intravasculaire selon l'une quelconque des revendications 1 à 8 et un cathéter ayant une lumière pour recevoir le fil pour enlever un corps étranger intravasculaire (200) dans celle-ci.
